# EUROPEAN PATENT APPLICATION

(11) **EP 0 719 552 A2**
(43) Date of publication of application: **03.07.1996**
(21) Application number: 95309435.6
(22) Date of filing: 22.12.1995
(51) Int. Cl.: A61K 31/20, A61K 31/19, A61K 31/12

(54) **Pharmaceutical composition comprising a quinone derivative or a hydroquinone thereof for treating dermatitis**

(30) Priority: 26.12.1994 JP 322994/94
(71) Applicant: Takeda Chemical Industries, Ltd., Osaka-shi, Osaka 541 (JP)
(72) Inventor: Ashida, Yasuko, Takatsuki, Osaka 569 (JP); Hiramoto, Akiko, Hirakata, Osaka 573 (JP); Shiraishi, Mitsuru, Amagasaki, Hyogo 661 (JP)
(74) Representative: Lewin, John Harvey

(57) **Abstract**

To provide a pharmaceutical composition for anti-atopic dermatitis exhibiting excellent therapeutic effect against dermatitis, especially atopic dermatitis

A pharmaceutical composition for dermatitis comprising as an active ingredient a quinone derivative of the formula: 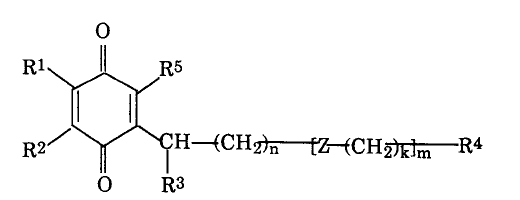 wherein
R¹ and R² are independently are a hydrogen atom, methyl or methoxy group, or R¹ and R² are linked together to form -CH=CH-CH=CH-;
R³ is an aliphatic, aromatic or heterocyclic group which may be substituted;
R⁴ is a hydrogen atom, methyl group, an optionally substituted hydroxyl group or a carboxyl group which may be esterified or amidated;
R⁵ is a hydrogen atom or methyl group;
Z is a group selected from the group consisting of -C≡C-, -CH=CH-, 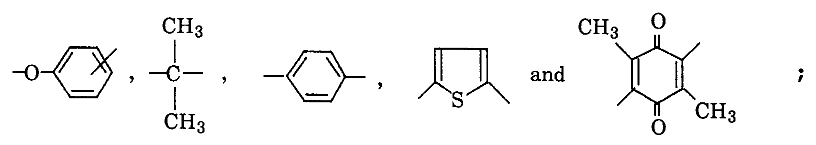 n is an integer of 0 to 15;
m is an integer of 0 to 3; and
k is an integer of 0 to 5,
provided that in the case of m being 2 or 3, Z and k can vary arbitrarily within the bracketed repeating unit, or a hydroquinone derivative thereof, or a salt thereof.

## Description

The present invention relates to a pharmaceutical composition for dermatitis, especially atopic dermatitis comprising a quinone derivative or a hydroquinone derivative thereof or a salt thereof exhibiting thromboxane A₂ receptor-antagonizing activity and antioxidant activity.

Atopic dermatitis is a disease underlain by an immune response in which eruption is caused by environmental factors, such as mites, food and stress, in addition to atopic predispositions, such as elevated serum IgE levels and skin dysfunction. It is held that abnormal production of IgE antibodies is due mainly to increased Th2-like cell number and function, accentuated IL-4 production being an exacerbating factor.

Among the therapeutic drugs for atopic dermatitis are steroids (external preparations) and anti-allergic and anti-histaminic drugs (oral preparations). Because oral preparations fail to achieve satisfactory effect when used alone, external steroids are the mainstream of atopic dermatitis chemotherapy. However, external steroids are very potent, causing adverse effects, including steroidal dermatopathy, with potential exacerbation upon withdrawal from medication, and for that reason, their use as medication is difficult. Atopic patients, in comparison with normal humans, have more activated Th2-like cells, with infiltration of activated lymphocytes, macrophages, eosinophils etc. in the epithelium and the upper layer of the dermis, and are likely to show immune response. Also, elevated blood eosinophil levels are often noted. Of the cytokines produced by Th2-like cells, IL-4 promotes IgE antibody production, while IL-5 promotes eosinophil differentiation, proliferation and migration and extending eosinophil life span. Therefore, drugs that suppress Th2-like cell function have the potential for improving atopic diseases [Saishin-Igaku, Vol. 49, pp. 40-55, 1994; Igaku no Ayumi, Vol. 168, pp. 731-735, 1994; Mebio, Vol. 19, 1992; Chiryogaku 8, Vol. 26, 1992].

For example, Japanese Published Unexamined Patent Application S61-44840/1986 (USP5180742) and S63-101322/1988 disclose that quinone derivatives exhibiting thromboxane A₂ synthase-inhibiting activity, thromboxane A₂ receptor-antagonizing activity and antioxidant activity are useful as anti-allergic agents, but there is no description of their applicability as pharmaceutical composition for treating or preventing dermatitis, especially atopic dermatitis.

The present invention provides a pharmaceutical composition for treating or preventing dermatitis, especially anti-atopic dermatitis comprising a quinone derivative or a hydroquinone derivative thereof or a salt thereof ehibiting thromboxane A₂ receptor-antagonizing activity and antioxidant activity.

The present invention relates to (1) a pharmaceutical composition for treating or preventing dermatitis which comprises a quinone derivative of the formula: 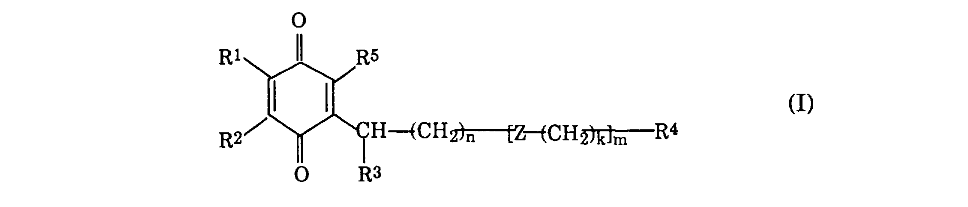 wherein
R¹ and R² are independently a hydrogen atom, methyl or methoxy group, or R¹ and R² are linked together to form -CH=CH-CH=CH-;
R³ is an aliphatic, aromatic or heterocyclic group which may be substituted;
R⁴ is a hydrogen atom, methyl group, an optionally substituted hydroxyl group or a carboxyl group which may be esterified or amidated;
R⁵ is a hydrogen atom or methyl group;
Z is a group selected from the group consisting of -C≡C-, -CH=CH-, 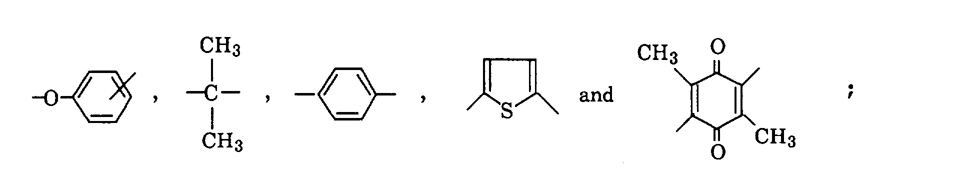
n is an integer of 0 to 15;
m is an integer of 0 to 3; and
k is an integer of 0 to 5,
provided that in the case of m being 2 or 3, Z and k can vary arbitrarily within the bracketed repeating unit,
or a hydroquinone derivative thereof,
or a salt thereof (hereinafter simply called compound (I)),
(2) a pharmaceutical composition in (1) above which is for treating or preventing atopic dermatitis,
(3) a pharmaceutical composition according to claim 1, wherein R¹ and R² are independently methyl or methoxy group, or R¹ and R² are linked together to form -CH=CH-CH=CH-,
(4) a pharmaceutical composition according to claim 1, R³ is a C₆₋₁₀ aryl group or a 5- or 6-membered heterocyclic group which may be substituted,
(5) a pharmaceutical composition according to claim 1, wherein R5 is methyl group,
(6) a pharmaceutical composition according to claim, wherein m is 0,
(7) a pharmaceutical composition in (1) above, wherein
   R¹ and R² are independently methyl or methoxy group, or R¹ and R² are linked together to form -CH=CH-CH=CH-;
   R3 is a phenyl group, naphthyl group, pyridyl group or thienyl group which may be substituted;
   R⁴ is a hydrogen atom, an optionally substituted hydroxyl group, or a carboxyl group which may be esterified or amidated;
   R⁵ is methyl group;
   n is an integer of 0 to 15;
   m is 0; and the dermatitis is atopic dermatitis,
(8) a pharmaceutical composition in (1) above, wherein
   R³ is a C₁₋₆ alkyl group, a C₂₋₄ alkenyl group, a C₃₋₇ cycloalkyl group, a C₆₋₁₀ aryl group or a 5- to 6-membered heterocyclic group which contains at least one hetero atom selected from the group consisting of oxygen atom, sulfur atom and nitrogen atom, wherein the C₆₋₁₀ aryl group and the heterocyclic group may have methylenedioxy group or trimethylene group, or may be substituted with 1 to 5 substituents selected from the group consisting of hydroxyl group, halogen atom, C₁₋₆ alkyl group, C₁₋₆ alkoxy group, acetyl group, phenyl group, p-tolyl group, m-tolyl group, pyridyl group, 3-pyridylmethyl group, benzoyl group, 1-imidazolyl group and 1-imidazolylmethyl group; the C₁₋₆ alkyl group and the C₂₋₄ alkenyl group may be subsituted with 1 to 5 substituents selected from the group consisting of hydroxyl group and halogen atom; and the C₃₋₇ cycloalkyl group may be substituted with 1 to 5 substituents selected from C₁₋₆ alkyl group;
   R⁴ is hydrogen atom, methyl group, hydroxyl group, C₁₋₆ alkoxy-C₁₋₆ alkoxy group, nitric acid group, aminocarbonyloxy group, mono- or di-C₂₋₇ alkylaminocarbonyloxy group, C₇₋₁₁ arylaminocarbonyloxy group, morpholinocarbonyloxy group, thiomorpholinocarbonyloxy group, piperidinocarbonyloxy group, C₁₋₆ alkoxy group, C₆₋₁₀ aryloxy group, C₇₋₁₂ aralkyloxy group, C₂₋₅ alkanoyloxy group, carboxyl group, C₂₋₈ alkoxycarbonyl group, C₇₋₁₂ aryloxycarbonyl group, C₈₋₁₃ aralkyloxycarbonyl group, aminocarbonyl group which may be substituted with 1 or 2 substituents selected from the group consisting of C₁₋₆ alkyl group, C₆₋₁₀ aryl group, C₇₋₁₂ aralkyl group and hydroxyl group, or,
      morpholinocarbonyl, thiomorpholinocarbonyl, piperidinocarbonyl, piperazinocarbonyl or pyrrolidinocarbonyl group which may be substituted with 1 to 3 substituents selected from the group consisting of C₁-₆ alkyl, C₆₋₁₀ aryl, C₇₋₁₃ aralkyl, amino carbonyl, 6-(9-β-D-ribofuranosido)adenyl and 4-amino-6,7-dimethoxyquinazolinyl group,
   (9) a pharmaceutical composition in (1) above, wherein both of R¹ and R² are methyl groups,
   (10) a pharmaceutical composition in (1) above, wherein R³ is an optionally substituted phenyl group,
   (11) a pharmaceutical composition in (1) above, wherein R³ is a phenyl group optionally substituted with at least one substituent selected from the group consisting of lower alkyl groups and halogen atom,
   (12) a pharmaceutical composition in (1) above, wherein R³ is phenyl, 3-fluorophenyl, 4-fluorophenyl, 3-methylphenyl, 4-methylphenyl, 2-thienyl or 3-thienyl group,
   (13) a pharmaceutical composition in (1) above,
   (14) a pharmaceutical composition in (1) above, wherein R⁴ is a carboxyl group or a hydroxyl group,
   (15) a pharmaceutical composition in (1) above, wherein n is an integer of 4 to 9,
   (16) a pharmaceutical composition in (1) above, wherein n is 5 and
   (17) a pharmaceutical composition in (1) above, wherein the quinone derivative is 7-(3,5,6-trimethyl-1,4-benzoquinon-2-yl)-7-phenylheptanoic acid.

In the above formula (I), R³ represents an aliphatic, aromatic or heterocyclic group which may be substituted. The said aliphatic group of R³ includes, for example, alkyl groups of 1 to 6 carbon atoms, such as methyl, ethyl, n-propyl, i-propyl, n-butyl, i-butyl and t-butyl, alkenyl groups of 2 to 4 carbon atoms, such as vinyl and allyl, and cycloalkyl groups of 3 to 7 carbon atoms, such as cyclopropyl, cyclobutyl, cyclopentyl, cyclohexyl and cycloheptyl;
the said aromatic group of R³ includes, for example, aryl groups of 6 to 10 carbon atoms, such as phenyl and naphthyl groups;
the said heterocyclic group of R³ includes, for example, 5-or 6-membered heterocyclic group which contains at least one hetero atom selected from the group consisting of oxygen atom, sulfur atom and nitrogen atom, and as the embodiment of the heterocyclic group, there may be mentioned, for example, thienyl (2-thienyl, 3-thienyl), furyl (2-furyl, 3-furyl) and pyridyl (2-pyridyl, 3-pyridyl) group.

These aromatic and heterocyclic groups may have methylenedioxy group or trimethylene group, or may be substituted with 1 to 5, preferably 1 to 3, of substituents in arbitrary positions on the rings, and such substituents include, for example, hydroxyl group, halogen atom, such as fluorine, chlorine and bromine, alkyl group of 1 to 6 carbon atoms, such as methyl, ethyl, propyl and isopropyl, alkoxy groups of 1 to 6 carbon atoms, such as methoxy and ethoxy, and acetyl, phenyl, p-tolyl, m-tolyl, pyridyl (2-pyridyl, 3-pyridyl), 3-pyridylmethyl, benzoyl, 1-imidazolyl and 1-imidazolylmethyl 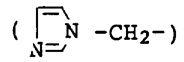 group.

When the aliphatic group is an alkyl group or an alkenyl group, it may be substituted with 1 to 5, preferably 1 to 3, of substituents selected from hydroxyl group and halogen atom (e.g. fluorine, chlorine, bromine), in any positions. When the aliphatic group is a cycloalkyl group, it may be substituted with 1 to 5, preferably 1 to 3, of substituents in any positions on the ring, and such substituents include alkyl groups of 1 to 6 carbon atoms, such as methyl and ethyl.

In the above formula (I), R⁴ represents a hydrogen atom, methyl group, an optionally substituted hydroxyl group or a carboxyl group which may be esterified or amidated.

Optionally substituted hydroxyl group represented by R⁴ include, for example, unsubstituted hydroxyl group, C₁₋₆ alkoxy-C₁₋₆ alkoxy, such as methoxymethoxy, nitric acid, aminocarbonyloxy, substituted aminocarbonyloxy (e.g., mono-or di-C₂₋₇ alkylaminocarbonyloxy, such as methylaminocarbonyloxy, ethylaminocarbonyloxy, dimethylaminocarbonyloxy, C₇₋₁₁ arylaminocarbonyloxy, such as phenylaminocarbonyloxy) and cyclic aminocarbonyloxy (e.g., morpholinocarbonyloxy, thiomorpholinocarbonyloxy, piperidinocarbonyloxy), alkoxy groups of 1 to 6 carbon atoms, such as methoxy, ethoxy, propoxy, isopropoxy, butoxy, isobutoxy, sec-butoxy, tert-butoxy, pentyloxy and hexyloxy, aryloxy groups of 6 to 10 carbon atoms, such as phenyloxy, 4-methylphenyloxy, α-naphthyloxy and β-naphthyloxy, aralkyloxys having 7 to 12 carbon atoms, such as benzyloxy, 4-methylbenzyloxy and phenethyloxy, and alkanoyloxy groups of 2 to 5 carbon atoms, such as acetyloxy, propionyloxy and butyryloxy.

The carboxyl group which may be esterified or amidated of R⁴ includes, carboxyl group, esterified carboxyl group or amidated carboxyl group.

The said esterified carboxyl group of R⁴ includes, for example, alkoxycarbonyl groups of 2 to 8 carbon atoms, such as methoxycarbonyl, ethoxycarbonyl, propoxycarbonyl, isopropoxycarbonyl, butoxycarbonyl, pentyloxycarbonyl, isopentyloxycarbonyl, tert-pentyloxycarbonyl and hexyloxycarbonyl, aryloxycarbonyl groups of 7 to 11 carbon atoms, such as phenyloxycarbonyl, 4-methylphenyloxycarbonyl, α-naphthyloxycarbonyl and β-naphthyloxycarbonyl, and aralkyloxycarbonyl groups of 8 to 13 carbon atoms, such as benzyloxycarbonyl, 4-methylbenzyloxycarbonyl and phenethyloxycarbonyl.

The said amidated carboxyl group of R⁴ may be substituted aminocarbonyl groups having its amino group substituted and also cyclic aminocarbonyl group.

The substituent for the amino group of such substituted aminocarbonyl group includes, for example, alkyl group of 1 to 6 carbon atoms, such as methyl, ethyl, propyl and butyl, aryl group of 6 to 10 carbon atoms (which may be substituted with further a substituent or substituents, such as hydroxyl, amino, nitro, halogen atoms (e.g. fluorine, chlorine, bromine), methyl and methoxy, in arbitrary positions on the ring), such as phenyl and naphthyl, (α-naphthyl, β-naphthyl), aralkyl groups of 7 to 12 carbon atoms, such as benzyl, 4-methylbenzyl and phenethyl, (α-phenethyl, β-phenethyl), 1-(α-naphthyl)ethyl and hydroxyl group. Specific examples of the amidated carboxyl group include, for example, aminocarbonyl(carbamoyl), mono- or di-alkylaminocarbonyl having 2 to 7 carbon atoms (e.g. methylaminocarbonyl, ethylaminocarbonyl, isopropylaminocarbonyl, dimethylaminocarbonyl), carboxymethylaminocarbonyl, aralkylaminocarbonyl of 8 to 13 carbon atoms (e.g. benzylaminocarbonyl, α-phenethylaminocarbonyl, β-phenethylaminocarbonyl, 1-(α-naphthyl)ethylaminocarbonyl), phenylaminocarbonyl, substituted phenylaminocarbonyl (p-hydroxyphenylaminocarbonyl, p-methylaminocarbonyl, m-chlorophenyl-aminocarbonyl), diphenylaminocarbonyl, hydroxyaminocarbonyl, N-hydroxy-N-methylaminocarbonyl, N-hydroxy-N-phenyl aminocarbonyl.

The said cyclic aminocarbonyl group includes, for example, morpholinocarbonyl, thiomorpholinocarbonyl, piperidinocarbonyl, piperazinocarbonyl and pyrrolidinocarbonyl group. The cyclic aminocarbonyl may be substituted with 1 to 3 substituents. The substituents includes unsubstituted- or substituted-C₁₋₆ alkyl (methyl, ethyl, propyl, pyrrolidinecarbonylmethyl, 4-fluorophenylcarbonylpropyl, etc.), unsubstituted- or substituted-C₆₋₁₀ aryl (phenyl, naphthyl, 2-methoxyphenyl, 4-methylphenyl, 4-bromophenyl, etc.), unsubstituted- or substituted-C ₇₋₁₃ aralkyl (benzyl, 2-phenylethyl, 4-fluorophenylmethyl, 4-methoxyphenylmethyl, 3,4,5-trimethoxyphenylmethyl, diphenylmethyl, etc.), aminocarbonyl, 6-(9-β-D-ribofuranosido)adenyl and 4-amino-6,7-dimethoxyquinazolinyl.

Among the amidated carboxyl groups, aminocarbonyl, carboxymethylaminocarbonyl and 4-ethylpiperazinocarbonyl are preferable, and carbamoyl is the most preferable.

In cases where R⁴ in the compound (I) is a carboxyl group, the compound (I) and its hydroquinone form may be a physiologically acceptable salt such as an alkali metal salt (e.g. sodium salt, potassium salt) or alkaline earth metal salt (e.g. calcium salt, magnesium salt).

In the above formula (I), n represents an integer of 0 to 15, 4 to 9 is preferable, and 5 is the most preferable.

In the above formula (I), m represents an integer of 0 to 3, 0 is preferable.

The hydroquinone of a compound of the formula (I) above is represented by the formula: 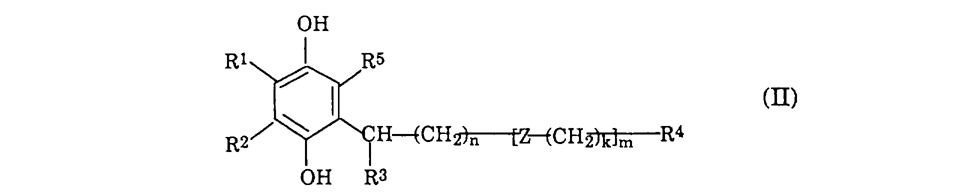 wherein the symbols are of the same meaning as defined above, hereinafter simply called compound (II).

Preferable example of the compound represented by the formula (I), or a hydroquinone derivative thereof, or a salt thereof, include a compound of the formula (I), wherein R¹ and R² are independently methyl or methoxy group, or R¹ and R² are linked together to form -CH=CH-CH=CH-; R³ is a phenyl, naphthyl, pyridyl or thienyl group which may be substituted; R⁴ is a hydrogen atom, an optionally substituted hydroxyl group, or a carboxyl group which may be esterified or amidated; n is an integer from 0 to 15; m is 0 and the dermatitis is atopic dermatitis, namely, a quinone derivative of the formula: 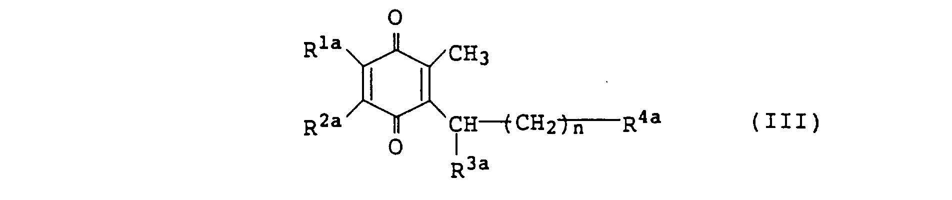 wherein R^{1a} and R^{2a} are independently methyl or methoxy group, or R^{1a} and R^{2a} are linked together to form -CH=CH-CH=CH-; R^{3a} is a phenyl, naphthyl, pyridyl or thienyl group which may be substituted; R^{4a} is a hydrogen atom, an optionally substituted hydroxyl group, or a carboxyl group which may be esterified or amidated; n is an integer from 0 to 15, hereinafter simply called compound (III), or a hydroquinone thereof, or a salt thereof, the most preferable example is 7-(3,5,6-trimethyl-1,4-benzoquinon-2-yl)-7-phenylheptanoic acid.

In the above formula (III), R^{3a} represents a phenyl, naphthyl, pyridyl or thienyl group which may be substituted. Examples of the naphthyl group for R^{3a} include 1-naphthyl or 2-naphthyl, examples of the pyridyl group for R^{3a} include 2-pyridyl, 3-pyridyl or 4-pyridyl, and examples of the thienyl group for R^{3a} include 2-thienyl or 3-thienyl. Phenyl or and thienyl is preferably, phenyl is most preferebly. The phenyl, naphthyl, pyridyl or thienyl for R^{3a} may have methylenedioxy or may be substituted with 1 to 3 substituents at any possible positions on their ring. Such substituents include lower alkyl groups of 1 to 4 carbon atoms, such as methyl, ethyl, propyl and isopropyl, lower alkoxy groups having 1 to 3 carbon atoms, such as methoxy and ethoxy, halogen atoms such as fluorine, chlorine and bromine, hydroxyl group and trifluoromethyl group. Lower alkyl groups or halogen atoms is preferably, fluorine or methyl is most preferably.

Preferable example of R^{3a} is phenyl group which may be substituted with at least one substituent selected from lower alkyl groups and halogen atoms, practically phenyl, 3-fluorophenyl, 4-fluorophenyl, 3-methylphenyl, 4-methylphenyl, 2-thienyl or 3-thienyl group is preferable, phenyl is the most preferable.

R^{4a} represents a hydrogen atom, an optionally substituted hydroxyl group, or a carboxyl group which may be esterified or amidated.

The optionally substituted hydroxyl group for R^{4a} is exemplified by the unsubstituted hydroxyl group, alkoxy groups of 1 to 6 carbon atoms, such as methoxy, ethoxy, propoxy, isopropoxy, butoxy, isobutoxy, sec-butoxy, tert-butoxy, pentyloxy and hexyloxy, aryloxy groups of 6 to 10 carbon atoms, such as phenyloxy, 4-methylphenyloxy, α-naphthyloxy and β-naphthyloxy, aralkyloxy groups having 7 to 10 carbon atoms, such as benzyloxy, 4-methylbenzyloxy and phenethyloxy, and alkanoyloxy groups of 2 to 5 carbon atoms, such as acetyloxy, propionyloxy and butyryloxy.

The carboxyl group for R^{4a}, which may be esterified or amidated, is exemplified by the free carboxyl group, esterified carboxyl groups and amidated carboxyl groups. Examples of esterified carboxyl groups include alkoxycarbonyl groups of 2 to 8 carbon atoms, such as methoxycarbonyl, ethoxycarbonyl, propoxycarbonyl, isopropoxycarbonyl, butoxycarbonyl, pentyloxycarbonyl, isopentyloxycarbonyl, tert-pentyloxycarbonyl and hexyloxycarbonyl, aryloxycarbonyl groups of 7 to 11 carbon atoms, such as phenyloxycarbonyl, 4-methylphenyloxycarbonyl, α-naphthyloxycarbonyl and β-naphthyloxycarbonyl, and aralkyloxycarbonyl groups of 8 to 11 carbon atoms, such as benzyloxycarbonyl, 4-methylbenzyloxycarbonyl and phenethyloxycarbonyl.

Amidated carboxyl groups for R^{4a} include the non-substitutional carbamoyl group, carbamoyl groups mono- or di-substituted at their amino group, and cyclic aminocarbamoyl groups. Examples of substituents for such mono- or di-substituted carbamoyl groups include alkyl groups of 1 to 4 carbon atoms, such as methyl, ethyl, propyl, isopropyl and butyl, aryl groups of 6 to 10 carbon atoms, such as phenyl, 4-methylphenyl, α-naphthyl and β-naphthyl, and aralkyl groups of 7 to 10 carbon atoms, such as benzyl, 4-methylbenzyl and phenethyl. Examples of cyclic aminocarbamoyl groups include morpholinocarbonyl, thiomorpholinocarbonyl, piperazinocarbonyl, 4-methylcarbonyl, pyrrolidinocarbonyl and pyrazinocarbonyl.

Preferable example of R^{4a} is carboxyl or hydroxyl group.

In cases where R^{4a} in the compound (III) is a carboxyl group, the compound (III) and its hydroquinone form may be a physiologically acceptable salt such as an alkali metal salt (e.g. sodium salt, potassium salt) or alkaline earth metal salt (e.g. calcium salt, magnesium salt).

In the above formula (III), n represents an integer of 0 to 15, 4 to 9 is preferable, 5 is the most preferable.

The hydroquinone of a compound of the formula (III) above is represented by the formula: 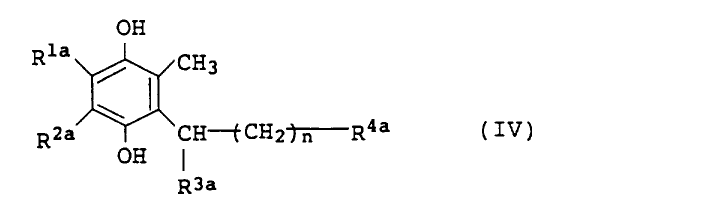 wherein the symbols are of the same meaning as defined above, hereinafter simply called compound (IV).

Compounds (I), (II), (III) and (IV), both used as active ingredients for the present invention, are described in Japanese Published Unexamined Patent Application S61-44840/1986 (USP5180742) or S63-101322/1988, and can be produced by the methods described therein.

The quinone derivative of the above formula (I) or (III) is biochemically convertible from and to the hydroquinone of the formula (II) or (IV) in vivo. These compounds can be considered equivalent both physiologically and pharmacologically.

In *in vitro* experimental systems, the onset of actions of compounds (I), (II), (III) and (IV) requires that R^{4a} is a free carboxyl group, while in *in vivo* experimental systems, anti-atopic dermatitis action is obtained when R⁴ or R^{4a} is a carboxyl group or a group convertible to a carboxyl group by oxidation (e.g., ω-oxidation, β-oxidation) or hydrolysis *in vivo.*

Preferable compounds of the above formulae (I), (II), (III) and (IV) include, those wherein R³ or R^{3a} is phenyl, 3- or 4-methylphenyl, 3- or 4-isopropylphenyl, 3- or 4-methoxyphenyl, 3- or 4-fluorophenyl, 2- or 3-thienyl, R⁴ or R^{4a} is a carboxyl group or hydroxymethyl, and the number (n) of methylene groups is an integer from 2 to 9, for use as active ingredients for the present invention.

The compound of the formula (I), (II), (III) or (IV) occurs in both the R- and S-configurations, the R-configuration is preferred from the viewpoint of drug efficacy in case that R³ or R^{3a} is an aryl group such as phenyl or naphthyl.

The compounds used as active ingredients for the present invention are of low toxicity. For example, when (±)-7-(3,5,6-trimethyl-1,4-benzoquinon-2-yl)-7-phenylheptanoic acid was orally administered at 1,000 mg/kg to 5 male ICR mice per group at 5 weeks of age, no deaths occurred during the 7-day observation period. The active ingredient compounds for the present invention are used as it is, or in pharmaceutical compositions (e.g., plasters, external preparations, tablets, granules, capsules (including soft capsules and microcapsules), liquids, injectable preparations, suppositories), together with commonly known pharmaceutically acceptable carriers, excipients etc., to treat and prevent dermatitis (e.g. atopic dermatitis, psoriasis, contact dermatitis, seborrheic dermatitis, nummular eczema, lichen simplex chronicus, ichthyosis vulgaris, pilar keratosis, scabies) for mammals (e.g. humans, dogs, cats, pigs), atopic dermatitis is preferably. Route of administration may be oral or non-oral. Depending on subject condition, route of administration, symptoms etc., it is advantageous that the daily dose of these compositions is usually about 10 to 500 mg, preferably about 10 to 200 mg, most preferably about 40 to 80 mg per administration, once or twice daily, in oral administration to an adult patient weighting 50 kg.

Compounds (I), (II), (III) and (IV), both used as active ingredients for the present invention, have a bulky group at the alpha-position carbon atom of the side chain of their quinone or hydroquinone nuclei, and have asymmetric centers. Although potent action is specific to either of the optical isomers formed due to presence of the asymmetric center, racemate compounds pose no problem in pharmaceutical efficacy.

With the bulky group at the alpha-position carbon atom of the side chain, the compounds used as active ingredients for the present invention are unlikely to undergo inactivating reaction via metabolism *in vivo,* therefore long maintaining effective blood levels and showing excellent effect at low doses.

The pharmaceutical composition for dermatitis, especially anti-atopic dermatitis in the present invention may be used in combination with an antifungal, antihistaminic or steroid, for instance. Combinations of compound (I), (II), (III) or (IV) and an antihistaminic or steroid can serve excellently as therapeutic/prophylactic agents for atopic dermatitis. Examples of antihistaminics include terfenadine, epinastine and ketotifen; examples of steroids include diflorasone diacetate, difluprednate and betamethasone valerate. These agents are used at ordinary doses.

When the pharmaceutical composition for dermatitis, especially anti-atopic dermatitis in the present invention is an external preparation, it may contain an antifungal agent in addition to antihistaminics and/or steroids. Examples of antifungal agents include azole-series antifungal agents such as oxiconazole, miconazole, econazole, isoconazole, sulconazole, tioconazole, croconazole, ketoconazole, neticonazole, latoconazole, aliconazole, itraconazole, omoconazole, fenticonazole, terconazole, clotrimazole and bifonazole; thiocarbamateseries antifungal agents such as tolnaftate, tolciclate and liranaftate (piritetrate); allylamine-series antifungal agents such as terbinafine and naftifine; benzylamineseries antifungal agents such as butenafine; morpholineseries antifungal agents such as amorolfine; iodopropargylseries antifungal agents such as haloprogin; pyrimidineseries antifungal agents such as ciclopirox olamine; benzamide-series antifungal agents such as exalamide; guanidinothiazole-series antifungal agents; antifungal antibiotics such as variotin, siccanin and pyrrolnitrin; and undecylenic acid-series antifungal agents such as undecylenic acid and esters thereof (e.g., phenyl-1-iodo10-undecynoate). The content ratio of the antifungal agent is set at an effective level against mycosis in the local medium for external preparations, e.g., 0.2 to 5% by weight, preferably about 0.5 to 3% by weight. The pharmaceutical composition for anti-atopic dermatitis in the present invention can also be administered as a bathing agent.

The effect of the present invention is hereinafter demonstrated by means of the following experimental examples.

### Experimental Example 1

### Effect on ascaris-immunized mouse splenocytes (in vivo)

Method: Female Balb/c mice at 9 weeks of age (purchased from Shizuoka Laboratory Animal Center, Japan) were intraperitoneally administered 40 µg/mouse DNP-ascaris (2,4-dinitrophenylated ascaris extract, produced by LSL) together with Freund's complete adjuvant (FCA, produced by Difco, USA), 3 weeks later, splenocytes were collected. The compound 1((±)-7-(3,5,6-trimethyl-1,4-benzoquino-2-yl)-7-phenylheptonoic acid) was suspended in 0.5% methyl cellulose (MC) and orally administered at 100 mg/kg/day 5 times weekly for 3 weeks, starting at the day before DNP-ascaris administration. After final oral administration, the splenocyte mixture was prepared from the spleens of 5 animals per group, and suspended in RPMI-1640 medium (produced by Bio Whittaker, Inc., USA) containing inactivated fetal bovine serum (10% v/v, produced by Difco, USA), 2-mercaptoethanol (50 µM), L-glutamine (2 mM) and gentamicin (20 µg/ml) to yield a single cell suspension. After cell number was set to 3.3 × 10⁶ cells/ml, DNP-ascaris was added to 96-well round-bottomed plates (produced by Sumitomo Bakelite Co., Ltd., Japan) at 12.5 µg/ml, and cultured at 37°C for 3 days in an incubator containing 5% carbon dioxide. After completion of cultivation, 3-(4,5-dimethylthiazol-2-yl)-2,5-diphenyltetrazolium bromide (MTT, produced by Dojin Kenkyujo, Japan) in PBS buffer was added to each well at 0.86 mg/ml, followed by 6 more hours of cultivation in the carbon dioxide incubator. To this culture, a 10% SDS-0.01N HCl mixture was added at 50 µl per well, followed by incubation in the carbon dioxide incubator for 18 hours, after which absorbance at 620 nm was determined. The results are shown in Table 1.

Next, DNP-ascaris was added to a 1.2 × 10⁶ cells/ml splenocyte suspension in each 96-well round-bottomed plate at 40 µg/ml and cultured at 37°C for 3 days in the 5% carbon dioxide incubator. After this culture was centrifuged at 200 x g for 5 minutes, the culture supernatant was collected, and, the amounts of IL-4 and IL-5 produced in each supernatant were determined by ELISA [mouse IL-4 ELISA kit (produced by Endogen, USA); mouse IL-5 ELISA kit (produced by Endogen, USA)]. The results are shown in Table 2.

**Table 1**

| Subjects Cells | DNP-ascaris Specific Growth Response, Relative Value^{a} |
|---|---|
| Splenocytes from mice administered 0.5% MC (not sensitized with antigen) | 1.00 |
| Splenocytes from mice administered 0.5% MC (sensitized with antigen) | 5.52 |
| Splenocytes from mice given compound 1 (sensitized with antigen) | 3.37 |

| | |
|---|---|
| a: Relative values were obtained, in comparison with absorbance for splenocytes from antigen-non-sensitized mice, taken as 1. | |

**Table 2**

| Subjects Cells | Cytokine Production, Relative Value^{b} | |
|---|---|---|
| | IL-4 | IL-5 |
| Splenocytes from mice adminitered 0.5% MC (not sensitized with antigen) | 1.00 | 1.00 |
| Splenocytes from mice adminitered 0.5% MC (sensitized with antigen) | 15.8 | 43.3 |
| Splenocytes from mice given compound 1 (sensitized with antigen) | 9.21 | 13.6 |

| | | |
|---|---|---|
| b: Relative values were obtained, in comparison with cytokine production by splenocytes from antigen-non-sensitized mice, taken as 1. | | |

### Experimental Example 2

### Effect on ascaris-immunized mouse splenocytes (in vitro)

Method: Female Balb/c mice at 9 weeks of age (purchased from Shizuoka Laboratory Animal Center, Japan) were intraperitoneally administered 40 µg/mouse DNP-ascaris (2,4-dinitrophenylated ascaris extract, produced by LSL), together with Freund's complete adjuvant (FCA, produced by Difco, USA), and, 3 weeks later, splenocytes were collected. Splenocyte mixture was prepared from the spleens and suspended in RPMI-1640 medium (produced by Bio Whittaker, Inc., USA) containing inactivated fetal bovine serum (10% v/v, produced by Difco, USA), 2-mercaptoethanol (50 µM), L-glutamine (2 mM) and gentamicin (20 µg/ml) to yield a single cell suspension. After cell number was set to 3.3 × 10⁶ cells/ml, DNP-ascaris was added to 96-well round-bottomed plates (produced by Sumitomo Bakelite Co., Ltd., Japan) containing a 25 µM test drug, at 12.5 µg/ml, and cultured at 37°C for 3 days in a 5% carbon dioxide incubator. After completion of cultivation, 3-(4,5-dimethylthiazol-2-yl)-2,5-diphenyl-tetrazolium bromide (MTT, produced by Dojin Kenkyujo, Japan) in PBS buffer was added to each well at 0.86 mg/ml, followed by cultivation in the carbon dioxide incubator for 6 hours. To this culture, a 10% SDS-0.01 N HCl mixture was added at 50 µl per well, followed by incubation in the carbon dioxide incubator for 18 hours, after which absorbance at 620 nm was determined.

Next, DNP-ascaris was added to a 1.2 × 10⁶ cells/ml splenocyte suspension in each 96-well round-bottomed plate containing a drug (25 µM), at 40 µg/ml, and cultured at 37°C for 3 days in the 5% carbon dioxide incubator. After this culture was centrifuged at 200 x g for 5 minutes, the culture supernatant was collected, and, the amount of IL-4 produced in each supernatant was determined by ELISA [mouse IL-4 ELISA kit (produced by Endogen, USA)]. The results are shown in Table 3.

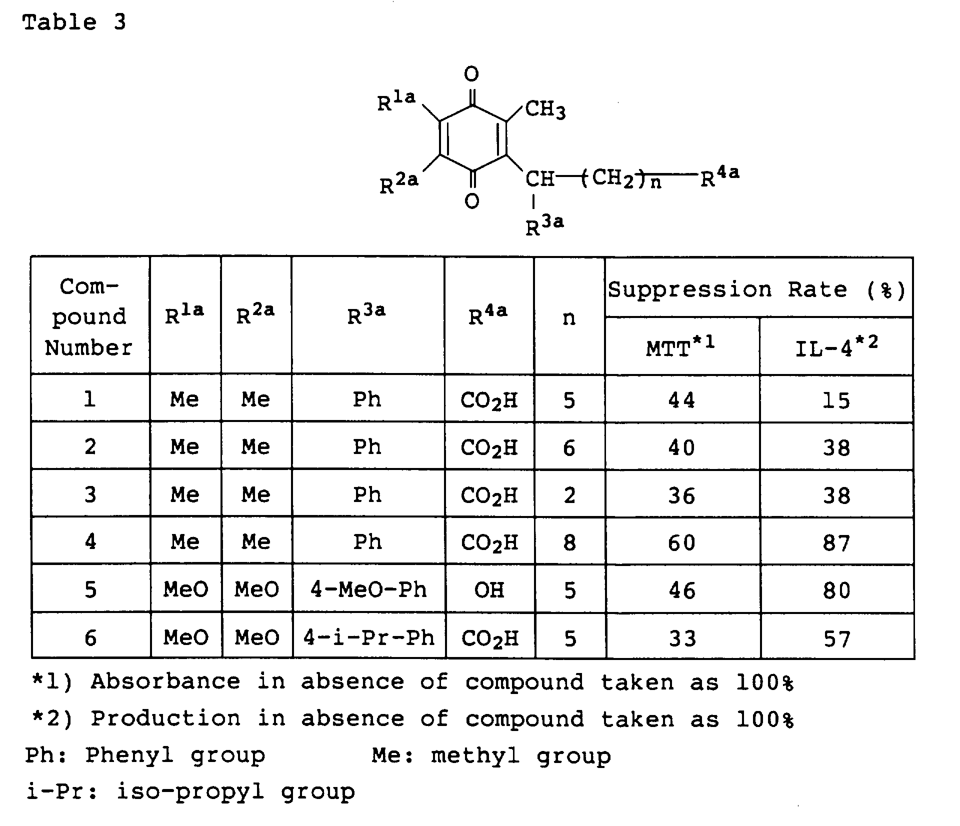

### Experimental Example 3

### Effect on ovalbumin-immunized mouse splenocytes

Method: Seven-week-old made Balb/c mice (Charles River Japan) were used. Mice were immunized i.p. with 1 ug ovalbumin (OVA, albumin chicken egg grade III; Sigma, USA) in aluminium hydroxide hydrate gel suspension (Alum, ISL Co., Ltd., Japan) twice at an unterval of a week. Two weeks after first immunization, spleens were asceptically removed, and single cell suspension was prepared in RPMI-1640 (Bio Whittaker, Inc., USA) containing 10% heat-inactivated fetal calf serum (FCS, Bio Whittaker, Inc., USA), 50 uM 2-mercaptoethanol, 2 mM L-glutamine, and 20 ug/ml gentamicin. Compound 1((+)-7-(3,5,6-trimethyl-1,4-benzoquino-2-yl)-7-phenylheptonoic acid) at 100 mg/kg suspended with 0.5% methyl cellulose (MC) was given orally in a volume of 10 ml/kg for two weeks from a day before first immunization. Splenocytes (3.3 × 10⁶ cells) were cultured with 10 ug/ml OVA in 125-ml wells of a 96-well round-bottomed tissue culture plate (Sumitomo Bakelite Co., Ltd., Japan) and were kept in a humidified incubation at 37°C in 5% CO₂ in air for three days.

To study the effect of drug on the proliferation, 50 ul of 3 mg/ml 3(4,5-dimethylthiazol-2-yl)-2,5-diphenyltetrazolium bromide (MTT, Dojinedo Labs., Japan) resolved in phosphate buffered saline (PBS) was added into each well for final 6 hours of culture. The culture was completed by added 50 ul of 10% SDS resolved into 0.1 N HCl solution and after kept in 37°C for 18 hours, the absorbance at 620 nm was measured. The results are shown in Table 4.

To study the effect of the drug on the cytokine production, after centrifuged at 200×g for 5 minutes, the supernatant of the culture well was removed and its IL-4 and IL-5 contents were measured by ELISA kits (Endogen, USA). The results are shown in Table 5.

**Table 4**

| Subjects Cells | OVA-Specific Growth Response, Relative Value^{a} |
|---|---|
| Splenocytes from mice administered 0.5% MC (not sensitized with antigen) | 1.00 |
| Splenocytes from mice administered 0.5% MC (sensitized with antigen) | 2.07 |
| Splenocytes from mice given compound 1 (sensitized with antigen) | 1.75 |

| | |
|---|---|
| a: Relative values were obtained, in comparison with growth response of splenocytes from antigen-non-sensitized mice, taken as 1 | |

.

**Table 5**

| Subject Cells | Cytokine Production, Relative Value^{b} | | | |
|---|---|---|---|---|
| | Exp. 1 | | Exp. 2 | |
| | IL-4 | IL-5 | IL-4 | IL-5 |
| Splenocytes from mice adminitered 0.5% MC (not sensitized with antigen) | 1.0 | 1.0 | 1.0 | 1.0 |
| Splenocytes from mice adminitered 0.5% MC (sensitized with antigen) | 24.7 | 7.3 | 14.2 | 31.8 |
| Splenocytes from mice given compound 1 (sensitized with antigen) | 7.7 | 4.8 | 6.94 | 21.7 |

| | | | | |
|---|---|---|---|---|
| b: Relative values were obtained, in comparison with cytokine production by splenocytes from antigen-non-sensitized mice, taken as 1. c: p<0.05 vs splenocytes from mice administered 0.5% MC (sensitized with antigen) Student t-test | | | | |

The results in Table 4 and Table 5 represent that compound 1 inhibits antigen-specific growth response and cytokine production in splenocytes from mice immunized with OVA.

### Experimental Example 4

### Effect on IL-5 mRNA expression in ovalbumin-immunized mouse splenocytes

Method: Eight-week-old male Balb/c mice (Charles River Japan) immunized i.p. with 1 ug OVA absorbed to 1 mg Alum twice at an unterval of a week. Two weeks after the first immunization, splenocytes were prepared in RPMI-1640 (BioWhittaker, Inc., USA) containing 10% heat-inactivated fetal bovine serum (FBS, Bio Whittaker, Inc., USA), 50 uM 2-mercaptoethanol, 2 mM L-glutamine, and 20 ug/ml gentamicin. Compound 1 ((±)-7-(3,5,6-trimethyl-1,4-benzoquino-2-yl)-7-phenylheptonoic acid) at 10 and 100 mg/kg suspended with 0.5% methyl cellulose (MC) was given orally in a volume of 20 ml/kg for two weeks from a day before first immunization. Splenocytes (3.3 × 10⁶ cells/ml) were cultured with 10 ug/ml OVA in tissue culture plate (Sumitomo Bakelite Co., Ltd., Japan). Four, seven and 24 hours later, splenocytes were collected with a cell scraper. Poly(A)+RNA was purified by a commercial mRNA purification kit (QuickPrep mRNA purification kit, Pharmacia Biotech) as recommended by the manufacturer. Poly(A)+RNA (1 υg) was reverse transcribed with MMLV reverse transcriptase and random primers included with the RT-PCR kit (Stratagene). Following the heat inactivation at 95°C for 10 min, one fifth of the cDNA mixtures was amplified by Ex Taq DNA polymerase (Takara Shuzoh, Japan) with the appropriate oligonucleatide primers (mouse IL-5 and β-actin amplimer set (Clontech). A DNA Thermal Cycler (Perkin Elmer) was used for 50 DNA amplification cycles, with a denaturation step at 95°C for 1 min, an annealing step at 55°C for 2 min, and an extension step at 72°C for 3 min. Twenty percent of the amplified DNA was analyzed by electrophoresis in a 2% GTG agarose gel. After staining with ethidium bromide, the DNA band was photographed under UV light. The data of the negatives were analyzed by NIH Image 1.55 software. The mRNA levels of β-actin was not affected by the treatment of compound 1 at the indicated times and therefore was used to standardize mRNA expression of the cytokine gene products. The ratio of densitometric value in cytokine PCR products to those of β-actin PCR product was employed to semi-quantitate relative levels of cytokine mRNA.

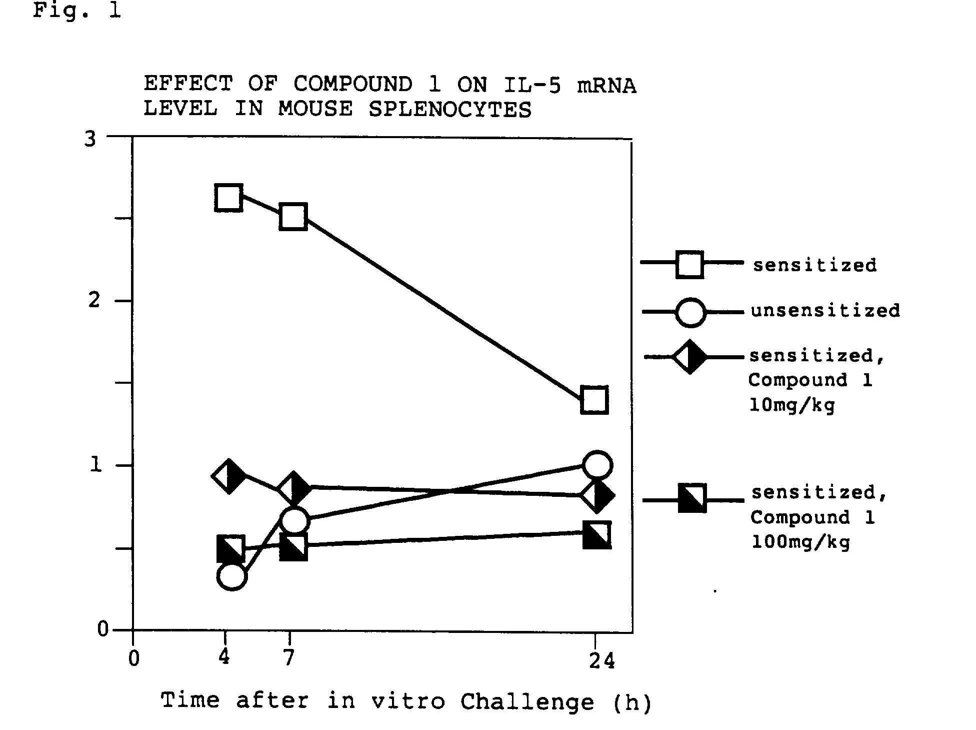

The result in Fig. 1 represents that compound 1 inhibits antigen-stimulated IL-5 mRNA expression in splenocytes from mice immunized with OVA.

| | |
|---|---|
| Preparation Example 1 (tablets) (40 mg tablet composition) (±)-7-(3,5,6-trimethyl-1,4-benzoquinon-2-yl)-7-phenylheptanoic acid | 40.0 mg |
| Hydroxypropyl cellulose | 3.6 mg |
| Magnesium stearate | 0.4 mg |
| Corn starch | 18.0 mg |
| Lactose | 58.0 mg |
| | Total 120.0 mg |

| | |
|---|---|
| Preparation Example 2 (tablets) (80 mg tablet composition) (±)-7-(3,5,6-trimethyl-1,4-benzoquinon-2-yl)-7-phenylheptanoic acid | 80.0 mg |
| Hydroxypropyl cellulose | 5.0 mg |
| Magnesium stearate | 0.5 mg |
| Corn starch | 24.5 mg |
| Lactose | 50.0 mg |
| | Total 160.0 mg |

| | |
|---|---|
| Preparation Example 3 (granules) (10% granule composition) (±)-7-(3,5,6-trimethyl-1,4-benzoquinon-2-yl)-7-phenylheptanoic acid | 100.0 mg |
| Hydroxypropyl cellulose | 30.0 mg |
| Carmellose calcium | 30.0 mg |
| Talc | 10.0 mg |
| Poloxamer-188 | 20.0 mg |
| Crystalline cellulose | 70.0 mg |
| Corn starch | 300.0 mg |
| Lactose | 440.0 mg |
| | Total 1000.0 mg |

| | |
|---|---|
| Preparation Example 4 (ointments) (White ointment) (±)-7-(3,5,6-trimethyl-1,4-benzoquinon-2-yl)-7-phenylheptanoic acid | 40 mg |
| Purified lanolin | 50 mg |
| Bleached beeswax | 50 mg |
| White petrolatum | 860 mg |
| | Total 1000 mg |

| | |
|---|---|
| (Simple ointment) (±)-7-(3,5,6-trimethyl-1,4-benzoqionon-2-yl)-7-phenylheptanoic acid | 40 mg |
| Beeswax | 330 mg |
| Vegetable oil | 630 mg |
| | Total 1000 mg |

| | |
|---|---|
| (Hydrophilic ointment) (±)-7-(3,5,6-trimethyl-1,4-benzoquinon-2-yl)-7-phenylheptanoic acid | 40 mg |
| White petrolatum | 250 mg |
| Stearyl alcohol | 200 mg |
| Propylene glycol | 120 mg |
| Polyxyethylene hardened castor oil 60 (HCO 60) | 40 mg |
| Glycerol monostearate | 10 mg |
| Methyl p-oxybenzoate | 1 mg |
| Propyl p-oxybenzoate | 1 mg |
| Purified water | 338 mg |
| | Total 1000mg |

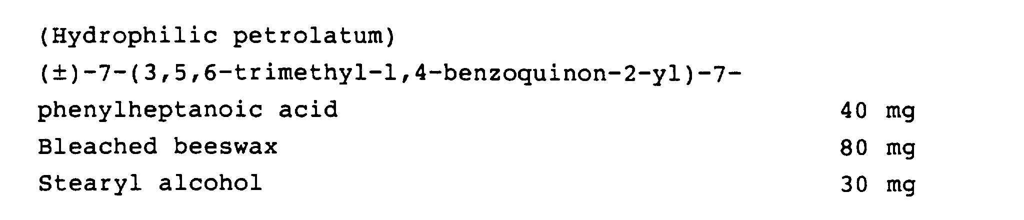

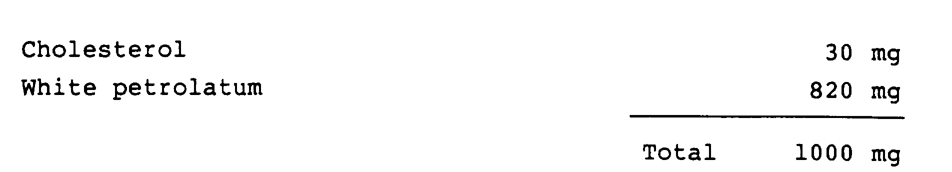

### Plasters

Prepared by applying one of the above-described ointment to cloth or adhesive tape

Pharmaceutical compositions comprising a compound of general formula (I), (II), (III) or (IV) exhibit excellent prophylactic/therapeutic action against dermatitis, especially atopic dermatitis.

## Claims

1. A pharmaceutical composition for treating or preventing dermatitis which comprises a quinone derivative of the formula: 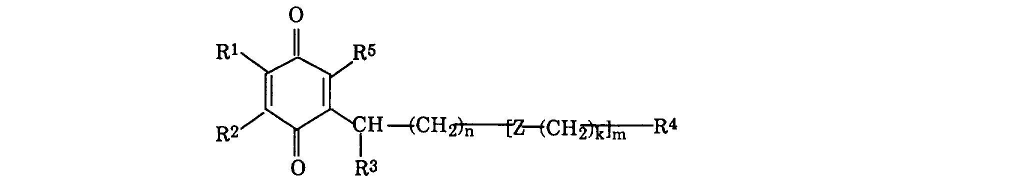 wherein
R¹ and R² are independently a hydrogen atom, methyl or methoxy group, or R¹ and R² are linked together to form -CH=CH-CH=CH-;
R³ is an aliphatic, aromatic or heterocyclic group which may be substituted;
R⁴ is a hydrogen atom, methyl group, an optionally substituted hydroxyl group or a carboxyl group which may be esterified or amidated;
R⁵ is a hydrogen atom or methyl group;
Z is a group selected from the group consisting of -C≡C-, -CH=CH-, 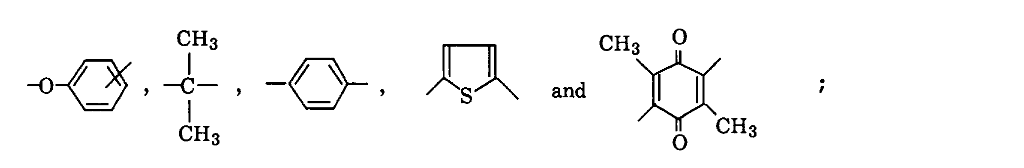
n is an integer of 0 to 15;
m is an integer of 0 to 3; and
k is an integer of 0 to 5,
provided that in the case of m being 2 or 3, Z and k can vary arbitrarily within the bracketed repeating unit, or a hydroquinone derivative thereof, or a salt thereof.

2. A pharmaceutical composition according to claim 1, which is for treating or preventing atopic dermatitis.

3. A pharmaceutical composition according to claim 1, wherein R¹ and R² are independently methyl or methoxy group, or R¹ and R² are linked together to form -CH=CH-CH=CH-.

4. A pharmaceutical composition according to claim 1, R3 is a C₆₋₁₀ aryl group or a 5- or 6-membered heterocyclic group which may be substituted.

5. A pharmaceutical composition according to claim 1, wherein R⁵ is methyl group.

6. A pharmaceutical composition according to claim 1, wherein m is 0.

7. A pharmaceutical composition according to claim 1, wherein
R¹ and R² are independently methyl or methoxy group, or R¹ and R² are linked together to form -CH=CH-CH=CH-;
R³ is a phenyl, naphthyl, pyridyl or thienyl group which may be substituted;
R⁴ is a hydrogen atom, an optionally substituted hydroxyl group, or a carboxyl group which may be esterified or amidated;
R⁵ is methyl group;
n is an integer of 0 to 15;
m is 0; and the dermetitis is atopic dermatitis.

8. A pharmaceutical composition according to claim 1, wherein
R³ is a C₁₋₆ alkyl group, a C₂₋₄ alkenyl group, a C₃₋₇ cycloalkyl group, a C₆₋₁₀ aryl group or a 5- or 6-membered heterocyclic group which contains at least one hetero atom selected from the group consisting of oxygen atom, sulfur atom and nitrogen atom, wherein the C₆₋₁₀ aryl group and the heterocyclic group may have methylenedioxy group or trimethylene group, or may be substituted with 1 to 5 substituents selected from the group consisting of hydroxyl group, halogen atom, C₁₋₆ alkyl group, C₁₋₆ alkoxy group, acetyl group, phenyl group, p-tolyl group, m-tolyl group, pyridyl group, 3-pyridylmethyl group, benzoyl group, trifluoromethyl group, 1-imidazolyl group and 1-imidazolylmethyl group; the C₁₋₆ alkyl group and the C₂₋₄ alkenyl group may be substituted with 1 to 5 of substituents selected from the group consisting of hydroxyl group and halogen atom; and the C₃₋₇ cycloalkyl group may be substituted with 1 to 5 substituents selected from C₁₋₆ alkyl group;
R⁴ is hydrogen atom, methyl group, hydroxyl group, C₁₋₆ alkoxy-C₁₋₆ alkoxy group, nitric acid group, aminocarbonyloxy group, mono- or di-C₂₋₇ alkylaminocarbonyloxy group, C₇₋₁₁ arylaminocarbonyloxy group, morpholinocarbonyloxy group, thiomorpholinocarbonyloxy group, piperidinocarbonyloxy group, C₁₋₆ alkoxy group, C₆₋₁₀ aryloxy group, C₇₋₁₂ aralkyloxy group, C₂₋₅ alkanoyloxy group, carboxyl group, C₂₋₈ alkoxycarbonyl group, C₇₋₁₁ aryloxycarbonyl group, C₈₋₁₃ aralkyloxycarbonyl group, aminocarbonyl group which may be substituted with 1 or 2 substituents selected from the group consisting of C₁₋₆ alkyl group, C₆₋₁₀ aryl group, C₇₋₁₂ aralkyl group and hydroxyl group, or,
morpholinocarbonyl, thiomorpholinocarbonyl,
piperidinocarbonyl, piperazinocarbonyl or
pyrrolidinocarbonyl group which may be substituted with 1 to 3 substituents selected from the group consisting of C₁₋₆ alkyl, C₆₋₁₀ aryl, C₇₋₁₃ aralkyl, aminocarbonyl, 6-(9-β-D-ribofuranoside)adenyl and 4-amino-6,7-dimethoxyquinazolinyl group.

9. A pharmaceutical composition according to claim 1, wherein both of R¹ and R² are methyl groups.

10. A pharmaceutical composition according to claim 1, wherein R³ is an optionally substituted phenyl group.

11. A pharmaceutical composition according to claim 1, wherein R³ is a phenyl group optionally substituted with at least one substituent selected from the group consisting of lower alkyl group and halogen atom.

12. A pharmaceutical composition according to claim 1, wherein R³ is phenyl, 3-fluorophenyl, 4-fluorophenyl, 3-methylphenyl, 4-methylphenyl, 2-thienyl or 3-thienyl group.

13. A pharmaceutical composition according to claim 1, wherein R³ is phenyl group.

14. A pharmaceutical composition according to claim 1, wherein R⁴ is a carboxyl group or a hydroxyl group.

15. A pharmaceutical composition according to claim 1, wherein n is an integer of 4 to 9.

16. A pharmaceutical composition according to claim 1, wherein n is 5.

17. A pharmaceutical composition according to claim 1, wherein the quinone derivative is 7-(3,5,6-trimethyl-1,4-benzoquinon-2-yl)-7-phenylheptanoic acid.

18. A method of treating or preventing dermatitis in a mammal which comprises administering to a mammal in need an effective amount of a compound of the formula: 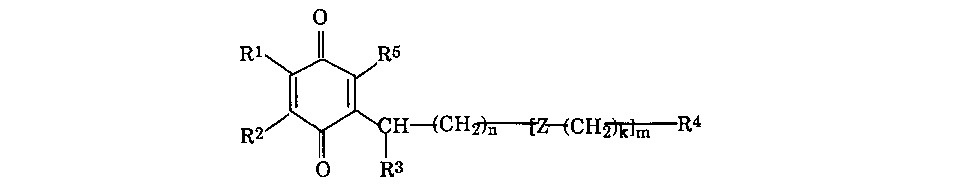 wherein
R¹ and R² are independently are a hydrogen atom, methyl or methoxy group, or R¹ and R² are linked together to form -CH=CH-CH=CH-;
R³ is an aliphatic, aromatic or heterocyclic group which may be substituted;
R⁴ is a hydrogen atom, methyl group, an optionally substituted hydroxyl group or a carboxyl group which may be esterified or amidated;
R⁵ is a hydrogen atom or methyl group;
Z is a group selected from the group consisting of -C≡C-, -CH=CH-, 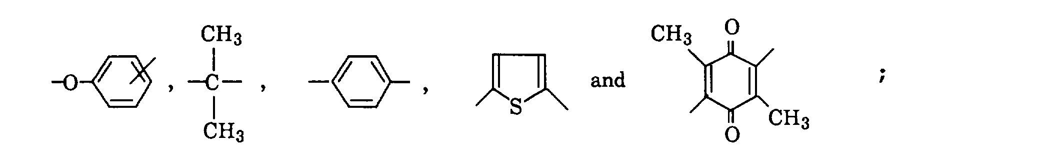
n is an integer of 0 to 15;
m is an integer of 0 to 3; and
k is an integer of 0 to 5,
provided that in the case of m being 2 or 3, Z and k can vary arbitrarily within the bracketed repeating unit, or a hydroquinone derivative thereof, or a salt thereof together with a pharmaceutically acceptable carrier or dilvent.

19. Use of a compound of the formula: 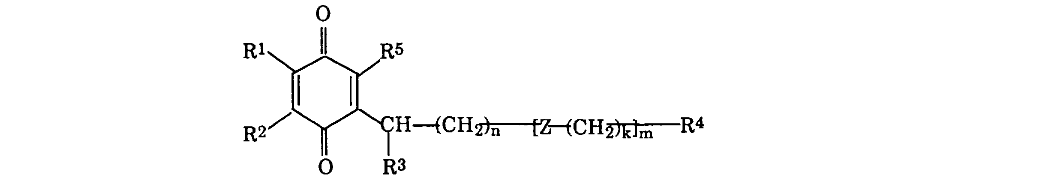 wherein
R¹ and R² are independently are a hydrogen atom, methyl or methoxy group, or R¹ and R² are linked together to form -CH=CH-CH=CH-;
R³ is an aliphatic, aromatic or heterocyclic group which may be substituted;
R⁴ is a hydrogen atom, methyl group, an optionally substituted hydroxyl group or a carboxyl group which may be esterified or amidated;
R⁵ is a hydrogen atom or methyl group;
Z is a group selected from the group consisting of -C≡C-, -CH=CH-, 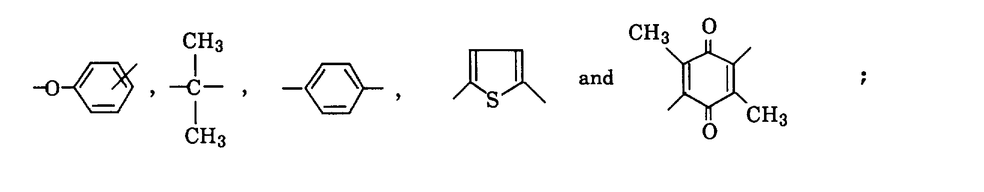
n is an integer of 0 to 15;
m is an integer of 0 to 3; and
k is an integer of 0 to 5,
provided that in the case of m being 2 or 3, Z and k can vary arbitrarily within the bracketed repeating unit, or a hydroquinone derivative thereof, or a salt thereof for preparing a pharmaceutical composition for treating or preventing dermatitis.
